# EUROPÄISCHE PATENTANMELDUNG

(11) **EP 1 972 333 A1**
(43) Veröffentlichungstag der Anmeldung: **24.09.2008**
(21) Anmeldenummer: 08102111.5
(22) Anmeldetag: 28.02.2008
(51) Int. Cl.: A61K 9/00, A61K 31/48, A61K 47/10

(54) **Stabilisierte wässrige Lösungen von Ergolinverbindungen**

(30) Priorität: 23.03.2007 DE 102007014947
(71) Anmelder: Axxonis Pharma AG, 10963 Berlin (DE)
(72) Erfinder: Schurad, Björn, 10119, Berlin (DE); Mottl, Harald, 9050, Appenzell (CH)
(74) Vertreter: Wablat, Wolfgang

(57) **Zusammenfassung**

Die vorliegende Erfindung betrifft stabilisierte wässrige Lösungen einer Ergolinverbindung der Formel I, 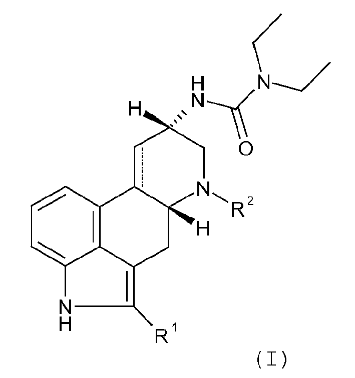 oder deren physiologisch verträglichem Salz oder Derivat, wobei R¹ ein H-Atom oder ein Halogenatom und R² eine Alkyl- oder Alkenylgruppe mit 1 bis 4 Kohlenstoffatomen undeine Einfach- oder Doppelbindung bedeutet, welche weiterhin 0.05 bis 90.00% mindestens eines sauerstoffhaltigen Cosolvenz enthalten. Ebenso betrifft die vorliegende Erfindung die Verwendung der erfindungsgemäß stabilisierten Lösungen zur Herstellung eines Mittels für die parenterale Behandlung von neurodegenerativen Erkrankungen oder Schädigungen des Gehirns.

## Beschreibung

Die vorliegende Erfindung betrifft stabilisierte wässrige Lösungen von Ergolinverbindungen oder deren physiologisch verträglichen Salzen oder Derivaten, in welchen die wässrige Lösung 0.05 bis 90.00% mindestens eines sauerstoffhaltigen Cosolvenz enthält. Ebenfalls betrifft die vorliegende Erfindung die Verwendung einer derartig stabilisierten wässrigen Lösung zur Herstellung eines Mittels für die parenterale Behandlung von neurodegenerativen Erkrankungen oder Schädigungen des Gehirns.

Heutzutage werden Ergolinverbindungen, wie beispielsweise Lisurid oder deren physiologisch verträgliche Salze, zur Behandlung von Patienten eingesetzt, die an der Parkinson-Krankheit (Stocchi, F. et al. (2002) Brain 125: 2058-2066), Meige-Syndrom (Ransmayr G. et al. (1988) Clinical Neuropharmacology 11: 68-76), Dystonien (Quinn N.P. et al. (1984) Neurology 34: 223) oder anderen schwerwiegenden Erkrankungen leiden. Die Verabreichung der notwendigen Wirkstofflösungen erfolgt intravenös, intramuskulär, transdermal oder subkutan.

Viele Ergolinverbindungen erweisen sich jedoch in gelöster Form (Lösungsmittel Wasser) als instabil. Gerade bei Vorliegen von 8α-Aminogruppen am Ringsystem, wie beispielsweise einer Diethylurea-Gruppe im Fall von Lisurid, sind die Verbindungen in Lösung extrem instabil und neigen zur schnellen Zersetzung (Hydrolyse).
Ein weiterer Nachteil liegt in der schlechten Löslichkeit natürlicher Ergotalkaloide sowie ihrer Salze und Derivate im Lösungsmittel Wasser.

Daher werden die entsprechenden Ergolinverbindungen, insbesondere Lisurid, momentan lediglich in lyophylisierter Form bereitgestellt. Diese muss vor der Verabreichung erst vom Patienten durch Zugabe einer entsprechenden Salzlösung rekonstituiert, d.h. wieder in eine flüssige Form gebracht werden.

Erst nach dieser Rekonstitution kann die Verabreichung an den Patienten erfolgen. Insbesondere bei Verwendung einer programmierbaren Mini- oder Mikropumpe muss die Lösung zunächst aber in eine spezielle Spritze überführt werden. Erst danach kann die abschließende Befüllung der Mini- oder Mikropumpe erfolgen.

Es ist daher leicht ersichtlich, dass die Art und die Vielzahl der durchzuführenden Schritte bei der Verwendung eines Lyophylisats diesen Vorgang extrem aufwendig und kompliziert gestaltet. Gerade für Patienten, welche aufgrund ihrer Erkrankung an Bewegungsstörungen (wie bei der Parkinson-Krankheit) oder an Dystonien leiden, erweist sich diese Vorgehensweise als äußerst schwierig.

Die momentan aus einem Lyophilisat rekonstituierte Lisurid-Lösung zeigt nur eine begrenzte Haltbarkeit, so dass eine Applikation dieser Lösung, beispielsweise mittels Mini- oder Mikropumpen, über ein größeres Zeitintervall ausgeschlossen werden muss.

Zusätzlich birgt die oben dargestellte, mehrstufige Vorgehensweise oft auch die Gefahr, dass die Lösungen nicht steril sind, da die Zubereitung durch den Anwender erfolgen muss. In solchen Fällen ist die Sicherheit des Patienten gefährdet, da die Verabreichung von nicht sterilen Lösungen unabsehbare Risiken mit sich bringt.

Aufgrund der genannten Nachteile der mehrstufigen Vorgehensweise wäre es wünschenswert, wenn eine Wirkstofflösung direkt bereitgestellt werden könnte.

Frühere Veröffentlichungen lehren insbesondere im Hinblick auf die Stabilität der Lösungen und die Löslichkeit von Ergotalkaloiden, dass die Nachteile in einem gewissen Umfang durch die Verwendung von Mischungen von Wasser und Alkoholen als Lösungsmittel überwunden werden könnten. Hierzu wird insbesondere der Einsatz von linearen monofunktionalen Alkoholen sowie von polyfunktionalen Alkoholen in Mengen von 60.00 bis 100.00% beschrieben. Im Rahmen der monofunktionalen Alkohole wird beispielsweise Ethanol verwendet. Als Vertreter der polyfunktionalen Alkohole kommen Propylenglykol, Glycerol oder Polyethylenglykol zur Anwendung (GB 2 062 568 A, DE 27 35 587 A1, BE 881967 A, GB 1 539 083 A).

Nachfolgende Veröffentlichungen erweitern diese Erkenntnisse auf Mischungen, welche aus Wasser und ausschließlich polyfunktionalen Alkoholen bei einem Alkoholgehalt von 10.00 bis 90.00% bestehen. Hierbei wird insbesondere die Verwendung von 25.00 bis 80.00% herausgestellt (DD 43 402 A; EP 0 101 879 A2). Die Anwendung bezieht sich in diesen Publikationen auf die rein orale Therapie.

Im Hinblick auf die Verträglichkeit für den Patienten ergeben sich generell Schwierigkeiten bei hohen Anteilen an Cosolventien wie den oben genannten Alkoholen. Gerade die Verwendung hoher Prozentsätze dieser Verbindungen führt dazu, dass die Lösungen die Anforderungen der entsprechenden Richtlinien nicht erfüllen.

Ebenfalls erhöht sich bei höheren Konzentrationen an Cosolvenz auch die Viskosität der Lösungen. Dies kann bei der Verabreichung, beispielsweise bei Verwendung einer Mini- oder Mikropumpe, zu Schwierigkeiten führen, da sich die Lösung schwerer pumpen lässt.

Die Beimischung entsprechender Alkohole in der Literatur beschränkt sich weiterhin auf die Verwendung bei natürlich auftretenden Ergotalkaloiden und ihren 9,10-Dihydroderivaten. Da diese Verbindungen keine 8α-Aminogruppierung aufweisen, sind sie allerdings bedeutend stabiler als Ergotalkaloide mit einer entsprechenden Aminogruppe in α-Stellung an Position 8 des Ringsystems. Eine -gegebenenfalls auch weiter substituierte Aminogruppe- neigt verstärkt zur Instabilität. Dies betrifft insbesondere die Diethylureagruppe -NHCON(C₂H₅)₂ wie beispielsweise im Falle des Lisurids, Tergurids, Bromergurids oder Protergurids oder ihrer pharmazeutisch verträglichen Salze und Ester. Aufgrund dieser erhöhten Instabilität wurden die oben dargelegten Stabilisierungsmethoden für diese Verbindungen als völlig ungeeignet angesehen.

Es ist daher die Aufgabe der vorliegenden Erfindung, wässrige stabilisierte Lösungen von Ergolinverbindungen der allgemeinen Formel I 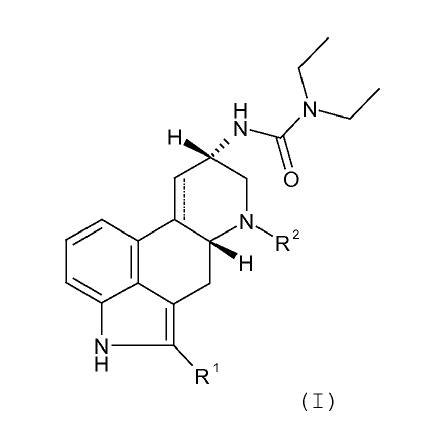 bereitzustellen.

Gelöst wird die Aufgabe durch eine stabilisierte wässrige Lösung einer Ergolinverbindung gemäß Anspruch 1, welche 0.05 bis 90.00% mindestens eines sauerstoffhaltigen Cosolvenz umfasst. Die %-Angaben im nachstehenden Text sind als Masse pro Volumen zu verstehen (m/V).

Weitere bevorzugte Ausführungsformen ergeben sich aus den Unteransprüchen.

In anderen Worten wird die Aufgabe durch eine stabilisierte wässrige Lösung gelöst, welche eine Ergolinverbindung der Formel I, 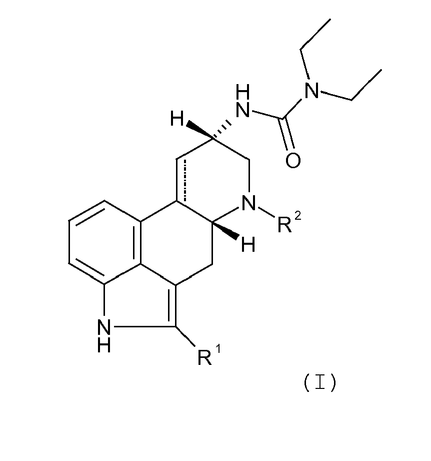 oder deren physiologisch verträgliches Salz oder Derivat, wobei R¹ ein H-Atom oder ein Halogenatom und R² eine Alkyl- oder Alkenylgruppe mit 1 bis 4 Kohlenstoffatomen undeine Einfach- oder Doppelbindung bedeutet, und weiterhin 0.05 bis 90.00% mindestens eines sauerstoffhaltigen Cosolvenz umfasst.

Bei dem mindestens einen sauerstoffhaltigen Cosolvenz handelt es sich bevorzugt um einen mehrwertigen Alkohol. Dieser kann vorzugsweise 2 bis 6 Kohlenstoffatome und mindestens zwei Hydroxylgruppen umfassen.

In einer bevorzugten Ausführungsform ist der mehrwertige Alkohol ausgewählt aus der Gruppe von 1,2-Ethandiol (Glykol), 1,2-Propandiol (Propylenglykol), 1,3-Propandiol, 1,2,3-Propantriol (Glycerol) oder 1,3-Butandiol.

Vorzugsweise ist das mindestens eine sauerstoffhaltige Cosolvenz ebenfalls ein Polyethylenglykol mit einer Molmasse von 200-35000 g/mol. Hierbei ist es besonders bevorzugt, dass das mindestens eine sauerstoffhaltige Cosolvenz ein Polyethylenglykol mit einer Molmasse von 200-4000 g/mol ist. Höchst bevorzugt handelt es sich bei dem mindestens einen sauerstoffhaltigen Cosolvenz um ein Polyethylenglykol mit einer Molmasse von 400 g/mol.

Die Konzentration des mindestens einen sauerstoffhaltigen Cosolvenz beträgt vorzugsweise 0.05 bis 20.00%. Besonders bevorzugt wird eine Konzentration des mindestens einen sauerstoffhaltigen Cosolvenz von 0.50 bis 9.50%.

In einer bevorzugten Ausführungsform ist das mindestens eine sauerstoffhaltige Cosolvenz Propylenglykol, wobei dessen Konzentration 0.05 bis 9.50% beträgt.

In einer weiteren bevorzugten Variante ist das mindestens eine sauerstoffhaltige Cosolvenz ein nichtionisches Detergens. Dieses ist vorzugsweise ausgewählt aus der Gruppe der Reaktionsprodukte von Polyethylenglykol, Ethylenoxid oder Polyglycerin mit Fettalkoholen, Alkoholen, hydriertem Ricinusöl, Fettsäuren, Hydroxyfettsäuren oder Alkylphenolen wie Nonylphenol oder deren Derivaten.

Dabei ist es bevorzugt, dass das nichtionische Detergens ausgewählt ist aus der Gruppe der Reaktionsprodukte aus Ethylenoxid und Castoröl, der Reaktionsprodukte von hydriertem Castoröl und Ethylenoxid, oder der Polyethylenglykol-15-hyroxystearate wie vorzugsweise Macrogol-15-hydroxystearat Ph. Eur. [European Pharmacopoeia] .

Besonders bevorzugt ist es, dass nichtionische Detergens aus der Gruppe von Reaktionsprodukten aus Ethylenoxid und Castoröl mit einem molaren Verhältnis von 20 - 60 : 1 oder Reaktionsprodukte von hydriertem Castoröl und Ethylenoxid mit einem molaren Verhältnis von 20 - 60 : 1 auszuwählen. Vorzugsweise ist das nichtionische Detergens dabei ausgewählt aus der Gruppe der Reaktionsprodukte aus Ethylenoxid und Castoröl mit einem molaren Verhältnis von 30 - 60 :1 oder der Reaktionsprodukte von hydriertem Castoröl und Ethylenoxid mit einem molaren Verhältnis von 30 - 60 :1. Höchst bevorzugt wird das nichtionische Detergens ausgewählt aus der Gruppe der Reaktionsprodukte aus Ethylenoxid und Castoröl mit einem molaren Verhältnis von 35 :1 oder der Reaktionsprodukte von hydriertem Castoröl und Ethylenoxid mit einem molaren Verhältnis von 40 :1 oder 60 : 1.

Weiterhin ist es bevorzugt, dass das nichtionische Detergens ausgewählt ist aus der Gruppe der Polyoxysorbitanfettsäureester, der Sorbitanfettsäureester oder der Polyoxyethylenpolyoxypropylene.

Vorzugsweise ist das nichtionische Detergens in einer Konzentration von 0.05 bis 90.00% enthalten. Besonders bevorzugt liegt es in einer Konzentration von 0.20 bis 20.00% vor. Höchst bevorzugt ist das nichtionische Detergens in einer Konzentration von 0.20 bis 10.00% enthalten.

Die Ergolinverbindung ist vorzugsweise ausgewählt aus der Gruppe von Lisurid, Tergurid, Protergurid und Bromergurid. Höchst bevorzugt als Ergolinverbindung ist Lisurid.

In einer bevorzugten Ausführungsform ist die Ergolinverbindung in Form ihres Salzes mit Schwefelsäure, schwefliger Säure, Phosphorsäure, phosphoriger Säure, Salpetersäure, salpetriger Säure, überchloriger Säure, Bromwasserstoffsäure, Salzsäure, Ameisensäure, Essigsäure, Propionsäure, Bernsteinsäure, Oxalsäure, Glukonsäure (Glykonsäure, Dextronsäure), Milchsäure, Apfelsäure, Weinsäure, Tartronsäure (Hydroxymalonsäure, Hydroxypropandisäure), Fumarsäure, Zitronensäure, Ascorbinsäure, Maleinsäure, Malonsäure, Hydroxymaleinsäure, Brenztraubensäure, Phenylessigsäure, ortho-Toluylsäure, metha-Toluylsäure, para-Toluylsäure, Benzoesäure, para-Aminobenzoesäure, para-Hydroxybenzoesäure, Salicylsäure, para-Aminosalicylsäure, Methansulfonsäure, Ethansulfonsäure, Hydroxyethansulfonsäure, Ethylensulfonsäure, para-Toluolsulfonsäure, Naphthylsulfonsäure, Naphthylaminsulfonsäure, Sufanilsäure, Camphorsulfonsäure, Chinasäure (Quininsäure), ortho-Methylmandelsäure, Hydrogenbenzolsulfonsäure, Pikrinsäure (2,4,6-Trinitrophenol), Adipinsäure, D-ortho-Tolylweinsäure oder einer Aminosäure enthalten.

Eine andere bevorzugte Variante ist das Vorliegen der Ergolinverbindung in Form ihres Salzes mit einer Aminosäure aus der Gruppe von Methionin, Tryptophan und Arginin.

Weiterhin ist es bevorzugt, dass die Ergolinverbindung in Form ihres Salzes mit einer säurehaltigen Aminosäure aus der Gruppe von Glutaminsäure und Asparaginsäure enthalten ist.

Eine höchst bevorzugte Ausführungsform ist dadurch gegeben, dass die Ergolinverbindung in Form ihres Salzes mit Maleinsäure enthalten ist.

Die Ergolinverbindung oder deren physiologisch verträgliches Salz oder Derivat ist vorzugsweise in einer Konzentration von 0.01 bis 25.00 mg/mL enthalten. Besonders bevorzugt liegt eine Konzentration der Ergolinverbindung oder deren physiologisch verträglichem Salz oder Derivat von 0.25 bis 10.00 mg/mL vor. Höchst bevorzugt ist es, dass die Ergolinverbindung oder deren physiologisch verträgliches Salz oder Derivat in einer Konzentration von 0.50 bis 3.00 mg/mL enthalten ist.

Weiterhin kann die stabilisierte wässrige Lösung anorganische und/oder organische Verbindungen zur Adjustierung der Osmolarität im Falle einer hypotonen Lösung und/oder zur Adjustierung des pH-Werts enthalten.

Vorzugsweise wird die stabilisierte Lösung in einer vorgefüllten Spritze oder in einer Carpule aufbewahrt.

Die Erfindung betrifft ebenfalls die Verwendung einer stabilisierten wässrigen Lösung zur Herstellung eines Mittels für die parenterale Behandlung von neurodegenerativen Erkrankungen oder Schädigungen des Gehirns. Dabei umfasst die stabilisierte wässrige Lösung eine Ergolinverbindung der Formel I, 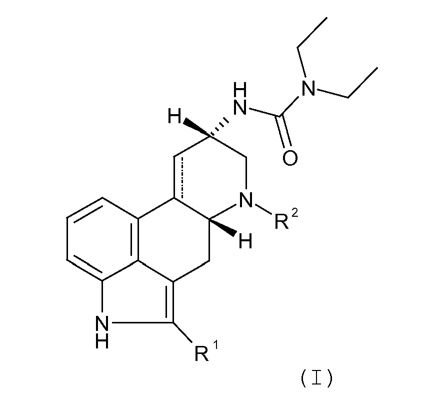 oder deren physiologisch verträgliches Salz oder Derivat, wobei R¹ ein H-Atom oder ein Halogenatom und R² eine Alkyl- oder Alkenylgruppe mit 1 bis 4 Kohlenstoffatomen undeine Einfach- oder Doppelbindung bedeutet, welche weiterhin 0.05 bis 90.00% mindestens eines sauerstoffhaltigen Cosolvenz aufweist.

Die parenterale Behandlung erfolgt vorzugsweise subkutan, intramuskulär, intravenös, transdermal oder durch eine in den Blutkreislauf oder das Gewebe implantierte Pumpe. Allgemein können im Rahmen der Pumpen Mini- oder Mikropumpen zum Einsatz kommen.

In einer Ausführungsform sind die neurodegenerativen Erkrankungen die Parkinson-Krankheit oder Dystonien.

Die Schädigung des Gehirns kann durch einen Schlaganfall oder eine traumatische Gehirnverletzung hervorgerufen worden sein.

Es hat sich überraschenderweise gezeigt, dass die erfindungsgemäßen Ergolinverbindungen oder deren physiologisch verträgliche Salze oder Derivate in Mischungen aus Wasser und mindestens einem sauerstoffhaltigen Cosolvenz mit einer Konzentration des Cosolvenz von 0.05 bis 90.00% gelöst werden können. Dabei können bevorzugt auch deutlich geringere Konzentrationen als die, welche im Stand der Technik für stabilere Ergotalkaloide beschrieben sind, eingesetzt werden. Insbesondere sind hier Konzentrationen von 0.05 bis 20.00%, besonders bevorzugt von 0.50 bis 9.50% möglich.

Da das mindestens eine Cosolvenz überraschenderweise die Löslichkeit des Wirkstoffs verbessert, kann das einem Patienten täglich zu verabreichende Volumen bei einer parenteralen dopaminergen Therapie deutlich reduziert werden.
Die Löslichkeit einer erfindungsgemäßen Modellverbindung konnte bei Verwendung eines erfindungsgemäßen Cosolvenz um einen Faktor von 1,5, insbesondere sogar um einen Faktor von 2 verbessert werden, wobei die Konzentration des erfindungsgemäßen Cosolvenz unter 10% lag und der pH-Wert der Lösung neutral war.

Die erhaltenen Lösungen weisen, insbesondere bei Verwendung der oben aufgeführten Alkohole, entgegen allen Erwartungen eine Haltbarkeit von mindestens 6 Monaten auf. Die Stabilisierung ist sogar noch deutlich verbessert im Hinblick auf Temperaturerhöhungen. Insgesamt ist im Vergleich zur unstabilisierten Lösung bereits bei Verwendung der nichtionischen Detergentien eine verbesserte Stabilität zu beobachten. Bei Verwendung der erfindungsgemäßen Alkohole, insbesondere bei Verwendung von Propylenglykol, ist der positive Effekt auf Löslichkeit und Stabilität der erfindungsgemäßen Ergolinverbindung am deutlichsten ausgeprägt.
Speziell im Hinblick auf die oben aufgeführten Alkohole, insbesondere für Propylenglykol, ergibt sich insgesamt ein zweifacher Vorteil: Zum einen wird die Löslichkeit des jeweiligen Wirkstoffes erhöht und zum anderen wird auch überraschenderweise die chemische Stabilität des jeweiligen Wirkstoffes deutlich verbessert.

Bei der Untersuchung von Proben konnte beispielsweise festgestellt werden, dass die Aufbewahrung bei 25°C unter Lichtschutz ohne Zusatz eines erfindungsgemäßen Cosolvenz innerhalb einer Zeitspanne von 180 Tagen bereits eine Zersetzung von 5% ergab. Bei der Aufbewahrung bei 6-8°C betrug die Zersetzung 0.5 bis 0.8%. Im Falle der Aufbewahrung bei 40°C waren bis zu 30% des Wirkstoffs zersetzt.
Im Gegensatz dazu führte die Beigabe eines erfindungsgemä-ßen Cosolvenz bei Aufbewahrung im Kühlschrank (6-8°C) für 180 Tage unter Lichtausschluß zu weniger als 0.5% Zerfall. Bei Raumtemperatur (25°C) ergab sich eine Zersetzung von im Mittel weniger als 0.5%.
Im Falle der Aufbewahrung bei 40°C waren bei Anwesenheit des erfindungsgemäßen Cosolvenz noch 89.8% des Wirkstoffs nachweisbar, d.h. es waren nur etwa 10% zersetzt.

Aufgrund der erfindungsgemäß stabilisierten Lösungen können den Patienten nun direkt Lösungen zur Verfügung gestellt werden. Dies stellt eine deutliche Erleichterung gegenüber den bisher verwendeten Lyophylisaten dar. Gleichzeitig werden auch die Probleme in Bezug auf die Sterilität behoben. Da überraschenderweise auch die Verwendung von sehr niedrigen Konzentrationen des Cosolvenz möglich ist, wird die parenterale Verabreichung deutlich erleichtert. Bei niedrigen Konzentrationen kann die Viskosität der Lösungen in einem gut zur Handhabung geeigneten Bereich gehalten werden. Weiterhin kann es mit den erfindungsgemäßen stabilisierten Lösungen vermieden werden, dass der Patient über längere Zeit unnötig hohen Dosen der verwendeten Alkohole ausgesetzt wird.

Beispielsweise beträgt die geschätzte Menge, welche als tägliche orale Dosis von Propylenglykol akzeptabel ist, 25 mg/kg kg (17th Report of the Joint FAO/WHO Expert Committee on Food Additives, 1974). Ebenso wurden Werte publiziert, welche Menge pro Tag bei subkutaner Verabreichung nicht akzeptabel ist. Daher wird es für die Verringerung von Sicherheitsrisiken für den Patienten als notwendig erachtet, ihn möglichst nur geringen Mengen der jeweiligen Alkohole auszusetzen.

Bei der Voraussetzung einer täglichen Menge von 2 mg Lisuridhydrogenmaleat ergibt sich bei Verwendung von 8.00% Propylenglykol und einer angenommenen Wirkstoffkonzentration von 2 mg Wirkstoff pro 3 mL Lösung eine Belastung des Patienten mit 240 mg Propylenglykol. Im Falle eines durchschnittlichen Körpergewichts von 70 kg beträgt die Dosis so weniger als 15% der für die orale Verabreichung akzeptablen Menge. Dies kann erfindungsgemäß durch weitere Erhöhung der Wirkstoffkonzentration und/oder Verringerung der Propylenglykolkonzentration optimiert werden.

Die erfindungsgemäße stabilisierte wässrige Lösung kann wie oben erwähnt anorganische und/oder organische Verbindungen zur Adjustierung der Osmolarität im Falle einer hypotonen Lösung und/oder des pH-Werts umfassen.

Durch Anpassung des pH-Wertes kann die Löslichkeit der erfindungsgemäßen Ergolinverbindungen oder deren physiologisch verträglichen Salze oder Derivate gesteigert werden. Eine Absenkung des pH-Wertes führt zu einer Erhöhung des Anteils der ionisierten Form der erfindungsgemäßen Ergolinverbindung und somit zu einer verbesserten Löslichkeit. Beispielsweise ergibt eine Absenkung des pH Wertes von pH 7 auf pH 6,2 bei einer repräsentativen erfindungsgemäßen Ergolinverbindung eine Verbesserung der Löslichkeit um einen Faktor von etwa 5, bei Absenkung von pH 7 auf pH 5,5 sogar um einen Faktor von etwa 20. Gegenläufig zur Löslichkeit verhält sich jedoch die Stabilität der erfindungsgemäßen Ergolinverbindungen. So konnte beobachtet werden, dass sich die Stabilität bei pH Absenkung in den sauren Bereich verschlechtert. Insgesamt hat es sich bei der Anpassung des pH-Werts als bevorzugt herausgestellt, einen pH-Wert im Bereich von 4.00 bis 8.00 einzustellen. Besonders bevorzugt ist ein pH-Bereich von 4.50 bis 7.50, höchst bevorzugt ist ein Bereich von 5.00 bis 7.00.

Zur Adjustierung des pH-Werts kann die stabilisierte wässrige Lösung ein Puffersystem aus der Gruppe von Citratpuffer, Carbonatpuffer, Phosphatpuffer oder Maleatpuffer umfassen. Bevorzugt als Puffersystem ist einen Citratpuffer.

Die Verträglichkeit in vivo der erfindungsgemäßen stabilisierten wässrigen Lösung einer erfindungsgemäßen Ergolinverbindung oder deren physiologisch verträglichem Salz oder Derivat kann durch Anpassung der Osmolarität optimiert werden. Dies ist der Fall bei einer erfindungsgemäßen im Vergleich zum Blut hypotonen Lösung. Dabei ist mit "hypoton" eine Lösung gemeint, welche einen geringeren osmotischen Druck als das menschliche Blut aufweist. Das Blut weist im Durchschnitt eine Osmolarität (= osmotischer Druck) von 290 mosmol/L (Milliosmol je Liter) auf. Bevorzugt wird dann durch Zugabe physiologisch verträglicher Hilfsstoffe auf eine Osmolarität von 250 bis 350 mosmol/L, höchst bevorzugt auf eine Osmolarität von 270 bis 320 mosmol/L eingestellt. Zur Adjustierung der Osmolarität wird Natriumchlorid bevorzugt. Natriumchlorid, welches einen physiologisch verträglichen Hilfsstoff darstellt, wird bevorzugt eingesetzt, um eine hypotone Lösung blutisoton einzustellen.

Für strukturverwandte Wirkstoffe, welche auf derselben Grundstruktur basieren, können ähnliche physikalischchemische Aktivitäten in vitro, in vivo sowie in klinischen Studien erwartet werden. Die Lisuridderivate Tergurid und Protergurid weisen Alkylsubstituenten am Stickstoff (N6) der Grundstruktur sowie eine Einfachbindung zwischen den Positionen 9 und 10 auf. Tatsächlich ergeben diese Verbindungen eine vergleichbare Löslichkeit in Wasser wie Lisurid. Die Löslichkeit des freien Protergurid beträgt beispielsweise 2.6 mg/100 mL in einer phosphatgepufferten Lösung (pH 7.4). Die Löslichkeit des freien Lisurids kann entsprechend auf 2.2 mg/100 mL abgeschätzt werden, wobei dieser Wert aus den Löslichkeitsdaten der jeweiligen Wirkstoffe/Wirkstoffsalze kalkuliert wurde (Zimmermann I (1983) International Journal of Pharmaceutics 13: 57-65).

Nachfolgend wird die Erfindung anhand von Beispielen näher erläutert.

### Beispiele

### Beispiel 1

Stabilität von Lisurid in gepufferten wässrigen Lösungen Es wurden Lösungen von Lisuridhydrogenmaleat mit einer Konzentration von jeweils 2 mg Wirkstoff pro 3 mL Lösung bezüglich ihrer temperaturabhängigen Stabilität untersucht. Die Lösungen wurden unter Verwendung eines Citratpuffersystems gepuffert, wobei pH-Werte des reinen Mediums von 5.1, 4.5 und 3.5 eingestellt wurden.
Es wurden wässrige Lösungen von Zitronensäure-Monohydrat und Trinatriumzitrat-Dihydrat (jeweils 0.53 mM) hergestellt.
Geeignetes Vermischen dieser Lösungen ergab wässrige Puffersysteme mit pH 4.5 bzw. 3.5. Nachdem 33.3 mg Lisuridhydrogenmaleat in einen skalierten 50 mL Kolben eingewogen worden waren, wurden jeweils 40 mL des entsprechenden Puffermediums hinzugegeben. Die Kolben wurden für mehrere Minuten geschüttelt und anschließend für mehrere Minuten Ultraschall ausgesetzt, bis der Wirkstoff vollständig gelöst war. Die Lösungen wurden auf Raumtemperatur abgekühlt und bis zur Markierung mit Puffermedium aufgefüllt. Abschließend wurde wieder für eine Minute geschüttelt.
Im Falle des stärker basischen Mediums (pH 5.1) wurde eine Vorratslösung hergestellt, welche Zitronensäure-Monohydrat (0.38 mM) und Trinatriumzitrat-Dihydrat (0.68 mM) enthielt. Diese wurde zum Lösen des Wirkstoffes eingesetzt. Prinzipiell wurde bidestilliertes Wasser zur Herstellung der Lösungen verwendet.
Die erhaltenen Lösungen wurden unter drei verschiedenen Bedingungen gelagert: im Kühlschrank bei 6 bis 8°C, bei Raumtemperatur (25°C) und bei einer erhöhten Temperatur von 40°C. Die Aufbewahrung erfolgte in verschlossenen Glasampulen, welche mit Aluminiumfolie als Lichtschutz umwickelt waren.
Nach angemessenen Zeitintervallen wurden Proben mittels einer spezifischen Umkehrphasen-HPLC-Methode mit UV-Detektion analysiert (Laufzeit 30 min, Retentionszeit des Lisurids ~13 min). Es konnte beobachtet werden, dass der Zerfall des Wirkstoffs von Temperatur und pH-Wert abhing. Der Zerfall erfolgte verstärkt bei hohen Temperaturen und hohen Konzentrationen an Oxoniumionen.
In jedem der Fälle war bei der Aufbewahrung bei Raumtemperatur innerhalb einer Zeitspanne von 180 Tagen bereits eine Zersetzung von 5% festzustellen. Bei der Aufbewahrung im Kühlschrank betrug die Zersetzung lediglich etwa 0.7%. Im Falle der Aufbewahrung bei 40°C waren bis zu 30% des Wirkstoffs zersetzt.
Alle Lösungen, welche bei 40°C aufbewahrt worden waren, wiesen nach 30 Tagen eine signifikante gelbe Färbung auf, welche sich nach weiteren 60 Tagen bis hin zu Braun verdunkelte.

**Tabelle 1: Reinheitsdaten für Lisuridhydrogenmaleat, gelöst in wässrigen citratgepufferten Lösungen.**

| | | | | | | | | |
|---|---|---|---|---|---|---|---|---|
| Die Daten sind in Prozent der Lisuridpeakfläche zur totalen Peakfläche des Chromatogramms angegeben. "SD" steht für Standard-Abweichung. | | | | | | | | |

| **Kühlschrank (6-8°C)** | | | **Raumtemperatur (25°C)** | | | **Erhöhte Temperatur (40°C)** | | |
|---|---|---|---|---|---|---|---|---|
| **pH** | | | **pH** | | | **pH** | | |
| **5.1** | | | **5.1** | | | **5.1** | | |

| **t [d]** | **Mittlere Fläche** | **Fläche SD** | **t [d]** | **Mittlere Fläche** | **Fläche SD** | **t [d]** | **Mittlere Fläche** | **Fläche SD** |
|---|---|---|---|---|---|---|---|---|
| 0 | 100,00 | | 0 | 100,00 | | 0 | 100,00 | |
| 7 | 99,90 | 0,05 | 7 | 99,79 | 0,04 | 7 | 98,86 | 0,06 |
| 30 | 99,72 | 0,03 | 30 | 99,36 | 0,03 | 30 | 94,71 | 0,26 |
| 90 | 99,59 | 0,02 | 90 | 98,64 | 0,14 | 90 | 90,99 | 0,37 |
| 180 | 99,29 | 0,04 | 180 | 95,45 | 1,17 | 180 | 87,98 | 0,61 |
| pH | | | pH | | | pH | | |
| 4.5 | | | 4.5 | | | 4.5 | | |

| **t [d]** | **Mittlere Fläche** | **Fläche SD** | **t [d]** | **Mittlere Fläche** | **Fläche SD** | **t [d]** | **Mittlere Fläche** | **Fläche SD** |
|---|---|---|---|---|---|---|---|---|
| 0 | 99,79 | | 0 | 99,79 | | 0 | 99,79 | |
| 7 | 99,88 | 0,03 | 7 | 99,84 | 0,01 | 7 | 98,82 | 0,12 |
| 30 | 99,73 | 0,01 | 30 | 99,26 | 0,06 | 30 | 94,96 | 0,09 |
| 90 | 99,75 | 0,11 | 90 | 98,14 | 0,28 | 90 | 90,39 | 0,30 |
| 180 | 99,11 | 0,13 | 180 | 95,90 | 1,19 | 180 | 87,71 | 0,19 |
| pH | | | pH | | | pH | | |
| 3.5 | | | 3.5 | | | 3.5 | | |

| **t [d]** | **Mittlere Fläche** | **Fläche SD** | **t [d]** | **Mittlere Fläche** | **Fläche SD** | **t [d]** | **Mittlere Fläche** | **Fläche SD** |
|---|---|---|---|---|---|---|---|---|
| 0 | 99,87 | | 0 | 99,87 | | 0 | 99,87 | |
| 7 | 99,92 | 0,01 | 7 | 99,89 | 0,01 | 7 | 98,89 | 0,02 |
| 30 | 99,80 | 0,02 | 30 | 99,37 | 0,07 | 30 | 90,41 | 0,12 |
| 90 | 99,57 | 0,07 | 90 | 98,70 | 0,13 | 90 | 73,37 | 1,15 |
| 180 | 99,22 | 0,15 | 180 | 94,14 | 1,90 | 180 | 69,91 | 0,89 |

### Beispiel 2

### Zusatz von 9% Propylenglykol

Es wurde eine wässrige Lösung von Lisuridhydrogenmaleat untersucht, welche zusätzlich 9% Propylenglykol enthielt. Die Wirkstoffkonzentration wurde auf 2 mg pro 3 mL eingestellt. Es wurde kein Puffersystem hinzugegeben.
Das weitere Vorgehen umfasste das Einwiegen von 33.3 mg des Wirkstoffs in einen skalierten 50 mL Kolben, anschließende Zugabe des Propylenglykols (4.5g) sowie die Beigabe von 40 mL Wasser. Der Kolben wurde für mehrere Minuten geschüttelt und anschließend Ultraschall ausgesetzt, bis der Wirkstoff vollständig gelöst war. Der Kolben wurde auf Raumtemperatur abgekühlt und bis zur Markierung mit bidestilliertem Wasser aufgefüllt. Abschließend wurde zur Gewährleistung der Homogenität für eine weitere Minute geschüttelt.
Die Lösung wurde in durchsichtige Glasampullen von 1 ml Volumen abgefüllt. Diese wurden luftdicht verschlossen und zum Lichtschutz mit Aluminiumfolie umwickelt. Die Ampullen wurden jeweils bei 6 bis 8°C, 25°C und 40°C aufbewahrt.
Die HPLC-Analyse ergab, dass diese Lösungen in einem signifikanten Maß stabiler waren als die Lösungen, welche ohne Zusatz von Propylenglykol hergestellt worden waren (vgl. Beispiel 1).
Nach Aufbewahrung im Kühlschrank für 180 Tage waren etwa 0.4% des Wirkstoffs zerfallen. Die bei Raumtemperatur gelagerten Proben ergaben ebenfalls einen verbliebenen Wirkstoffgehalt von 98.4% im Vergleich zur Ausgangskonzentration. Im Falle der Aufbewahrung bei 40°C waren lediglich etwa 10% des Wirkstoffs zersetzt.

Zusätzlich trat die Verfärbung der Lösungen deutlich verzögert ein im Vergleich zu den Lösungen aus Beispiel 1. Eine leichte gelbe Färbung wurde erst nach einer Lagerzeit von 90 Tagen bei erhöhter Temperatur beobachtet.

Ein weiterer Vorteil bei der Verwendung von Propylenglykol als gering dosiertes Additiv (weniger als 10%) lag in der verbesserten Löslichkeit des Wirkstoffs (Lisuridhydrogenmaleat). Entsprechend kann das einem Patienten täglich zu verabreichende Volumen nach Optimierung der Arzneistoffkonzentration bei einer parenteralen dopaminergen Therapie neurodegenerativer Erkrankungen deutlich reduziert werden.

**Tabelle 2: Reinheitsdaten für Lisuridhydrogenmaleat, gelöst in bidestilliertem Wasser unter Zusatz von 9% Propylenglykol.**

| | | | | | | | | |
|---|---|---|---|---|---|---|---|---|
| Die Daten sind in Prozent der Lisuridpeakfläche zur totalen Peakfläche des Chromatogramms angegeben. "SD" steht für Standard-Abweichung. | | | | | | | | |

| **Kühlschrank (6-8°C)** | | | **Raumtemperatur (25°C)** | | | **Erhöhte Temperatur (40°C)** | | |
|---|---|---|---|---|---|---|---|---|
| **t [d]** | **Mittlere Fläche** | **Fläche SD** | **t [d]** | **Mittlere Fläche** | **Fläche SD** | **t [d]** | **Mittlere Fläche** | **Fläche SD** |
| 0 | 99.80 | | 0 | 99.80 | | 0 | 99.80 | |
| 7 | 99.79 | 0.09 | 7 | 99.79 | 0.01 | 7 | 99.33 | 0.06 |
| 30 | 99.67 | 0.01 | 30 | 99.46 | 0.02 | 30 | 97.59 | 0.18 |
| 90 | 99.52 | 0.05 | 90 | 99.15 | 0.02 | 90 | 94.06 | 0.93 |
| 180 | 99.36 | 0.08 | 180 | 98.35 | 0.34 | 180 | 89.76 | 0.50 |

### Beispiel 3

### Zusatz von Cremophor ELP

Es wurde eine wässrige Lisuridhydrogenmaleatlösung, welche verschiedene Additive enthielt, im Hinblick auf ihre verbesserte Stabilität untersucht. Als Additive wurden Propylenglykol und Cremophor ELP in Konzentrationen zwischen 1.00 und 10.00% eingesetzt.
Die Herstellung der Lösungen erfolgte analog zur Vorgehensweise, wie sie in den Beispielen 1 und 2 angewandt wurde. Die Lösungen wurden im Dunkeln in Glasbehältern bei 60°C für eine Woche aufbewahrt.
Es wurde gefunden, dass Propylenglykol zu einer signifikant verbesserten Stabilität des Wirkstoffs im Vergleich zu Cremophor ELP führte. Der verbliebene Wirkstoffgehalt bei der Verwendung von Propylenglykol betrug 92.5%. Im Gegensatz dazu waren unter den gleichen Bedingungen bei Verwendung des Cremophors ELP 14,3-21.3% (1%, bzw. 10% Cremophor ELP) des Wirkstoffs zerfallen.
Dies weist ebenfalls auf die bessere Eignung von insbesondere Propylenglykol im Vergleich zu anderen Additiven hin, da sich insgesamt ein zweifacher Vorteil ergibt. Zum einen wird die Löslichkeit des jeweiligen Wirkstoffes erhöht und zum anderen wird auch überraschenderweise die chemische Stabilität des jeweiligen Wirkstoffes verbessert.

## Patentansprüche

1. Stabilisierte wässrige Lösung einer Ergolinverbindung der Formel I, 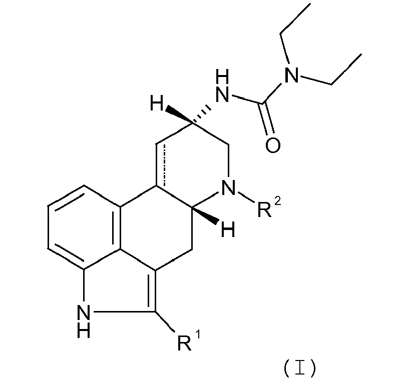 oder deren physiologisch verträglichem Salz oder Derivat, wobei R¹ ein H-Atom oder ein Halogenatom und R² eine Alkyl- oder Alkenylgruppe mit 1 bis 4 Kohlenstoffatomen und ----- eine Einfach- oder Doppelbindung bedeutet, **dadurch gekennzeichnet, dass** die wässrige Lösung weiterhin 0.05 bis 90.00% (m/V) mindestens eines sauerstoffhaltigen Cosolvenz umfasst.

2. Stabilisierte wässrige Lösung nach Anspruch 1, **dadurch gekennzeichnet, dass** das mindestens eine sauerstoffhaltige Cosolvenz ein mehrwertiger Alkohol ist.

3. Stabilisierte wässrige Lösung nach einem der Ansprüche 1 bis 2, **dadurch gekennzeichnet, dass** der mehrwertige Alkohol 2 bis 6 Kohlenstoffatome und mindestens zwei Hydroxylgruppen umfasst.

4. Stabilisierte wässrige Lösung nach einem der Ansprüche 1 bis 3, **dadurch gekennzeichnet, dass** der mehrwertige Alkohol ausgewählt ist aus der Gruppe von 1,2-Ethandiol, 1,2-Propandiol, 1,3-Propandiol, 1,2,3-Propantriol oder 1,3-Butandiol.

5. Stabilisierte wässrige Lösung nach einem der Ansprüche 1 bis 4, **dadurch gekennzeichnet, dass** das mindestens eine sauerstoffhaltige Cosolvenz ein Polyethylenglykol mit einer Molmasse von 200-35000 g/mol ist.

6. Stabilisierte wässrige Lösung nach einem der Ansprüche 1 bis 5, **dadurch gekennzeichnet, dass** das mindestens eine sauerstoffhaltige Cosolvenz ein Polyethylenglykol mit einer Molmasse von 200-4000 g/mol ist.

7. Stabilisierte wässrige Lösung nach einem der Ansprüche 1 bis 6, **dadurch gekennzeichnet, dass** das mindestens eine sauerstoffhaltige Cosolvenz ein Polyethylenglykol mit einer Molmasse von 400 g/mol ist.

8. Stabilisierte wässrige Lösung nach einem der Ansprüche 1 bis 7, **dadurch gekennzeichnet, dass** die Konzentration des mindestens einen sauerstoffhaltigen Cosolvenz 0.05 bis 20.00% beträgt.

9. Stabilisierte wässrige Lösung nach einem der Ansprüche 1 bis 8, **dadurch gekennzeichnet, dass** die Konzentration des mindestens einen sauerstoffhaltigen Cosolvenz 0.50 bis 9.50% beträgt.

10. Stabilisierte wässrige Lösung nach einem der Ansprüche 1 bis 9, **dadurch gekennzeichnet, dass** das mindestens eine sauerstoffhaltige Cosolvenz Propylenglykol ist und dessen Konzentration 0.05 bis 9.50% beträgt.

11. Stabilisierte wässrige Lösung nach einem der Ansprüche 1 bis 10, **dadurch gekennzeichnet, dass** das mindestens eine sauerstoffhaltige Cosolvenz ein nichtionisches Detergens ist.

12. Stabilisierte wässrige Lösung nach einem der Ansprüche 1 bis 11, **dadurch gekennzeichnet, dass** das nichtionische Detergens ausgewählt ist aus der Gruppe der Reaktionsprodukte von Polyethylenglykol, Ethylenoxid oder Polyglycerin mit Fettalkoholen, Alkoholen, hydriertem Ricinusöl, Fettsäuren, Hydroxyfettsäuren oder Alkylphenolen wie Nonylphenol oder deren Derivaten.

13. Stabilisierte wässrige Lösung nach einem der Ansprüche 1 bis 12, **dadurch gekennzeichnet, dass** das nichtionische Detergens ausgewählt ist aus der Gruppe der Reaktionsprodukte aus Ethylenoxid und Castoröl, der Reaktionsprodukte von hydriertem Castoröl und Ethylenoxid, oder der Polyethylenglykol-15-hyroxystearate.

14. Stabilisierte wässrige Lösung nach einem der Ansprüche 1 bis 13, **dadurch gekennzeichnet, dass** das Polyethylenglykol-15-hyroxystearat Macrogol-15-hydroxystearat Ph. Eur. ist.

15. Stabilisierte wässrige Lösung nach einem der Ansprüche 1 bis 14, **dadurch gekennzeichnet, dass** das nichtionische Detergens ausgewählt ist aus der Gruppe der Reaktionsprodukte aus Ethylenoxid und Castoröl mit einem molaren Verhältnis von 20 - 60 : 1 oder der Reaktionsprodukte von hydriertem Castoröl und Ethylenoxid mit einem molaren Verhältnis von 20 - 60 : 1.

16. Stabilisierte wässrige Lösung nach einem der Ansprüche 1 bis 15, **dadurch gekennzeichnet, dass** das nichtionische Detergens ausgewählt ist aus der Gruppe der Reaktionsprodukte aus Ethylenoxid und Castoröl mit einem molaren Verhältnis von 30 - 60 :1 oder der Reaktionsprodukte von hydriertem Castoröl und Ethylenoxid mit einem molaren Verhältnis von 30 - 60 :1.

17. Stabilisierte wässrige Lösung nach einem der Ansprüche 1 bis 16, **dadurch gekennzeichnet, dass** das nichtionische Detergens ausgewählt ist aus der Gruppe der Reaktionsprodukte aus Ethylenoxid und Castoröl mit einem molaren Verhältnis von 35 :1 oder der Reaktionsprodukte von hydriertem Castoröl und Ethylenoxid mit einem molaren Verhältnis von 40 :1 oder 60 : 1.

18. Stabilisierte wässrige Lösung nach einem der Ansprüche 1 bis 17, **dadurch gekennzeichnet, dass** das nichtionische Detergens ausgewählt ist aus der Gruppe der Polyoxysorbitanfettsäureester, der Sorbitanfettsäureester oder der Polyoxyethylenpolyoxypropylene.

19. Stabilisierte wässrige Lösung nach einem der Ansprüche 1 bis 18, **dadurch gekennzeichnet, dass** das nichtionische Detergens in einer Konzentration von 0.05 bis 90.00% enthalten ist.

20. Stabilisierte wässrige Lösung nach einem der Ansprüche 1 bis 19, **dadurch gekennzeichnet, dass** das nichtionische Detergens in einer Konzentration von 0.20 bis 20.00% enthalten ist.

21. Stabilisierte wässrige Lösung nach einem der Ansprüche 1 bis 20, **dadurch gekennzeichnet, dass** das nichtionische Detergens in einer Konzentration von 0.20 bis 10.00% enthalten ist.

22. Stabilisierte wässrige Lösung nach einem der Ansprüche 1 bis 21, **dadurch gekennzeichnet, dass** die Ergolinverbindung ausgewählt ist aus der Gruppe von Lisurid, Tergurid, Protergurid und Bromergurid.

23. Stabilisierte wässrige Lösung nach einem der Ansprüche 1 bis 22, **dadurch gekennzeichnet, dass** die Ergolinverbindung Lisurid ist.

24. Stabilisierte wässrige Lösung nach einem der Ansprüche 1 bis 23, **dadurch gekennzeichnet, dass** die Ergolinverbindung in Form ihres Salzes mit Schwefelsäure, schwefliger Säure, Phosphorsäure, phosphoriger Säure, Salpetersäure, salpetriger Säure, überchloriger Säure, Bromwasserstoffsäure, Salzsäure, Ameisensäure, Essigsäure, Propionsäure, Bernsteinsäure, Oxalsäure, Glukonsäure, Milchsäure, Apfelsäure, Weinsäure, Tartronsäure, Fumarsäure, Zitronensäure, Ascorbinsäure, Maleinsäure, Malonsäure, Hydroxymaleinsäure, Brenztraubensäure, Phenylessigsäure, ortho-Toluylsäure, metha-Toluylsäure, para-Toluylsäure, Benzoesäure, para-Aminobenzoesäure, para-Hydroxybenzoesäure, Salicylsäure, para-Aminosalicylsäure, Methansulfonsäure, Ethansulfonsäure, Hydroxyethansulfonsäure, Ethylensulfonsäure, para-Toluolsulfonsäure, Naphthylsulfonsäure, Naphthylaminsulfonsäure, Sufanilsäure, Camphorsulfonsäure, Chinasäure, ortho-Methylmandelsäure, Hydrogenbenzolsulfonsäure, Pikrinsäure, Adipinsäure, D-ortho-Tolylweinsäure oder einer Aminosäure enthalten ist.

25. Stabilisierte wässrige Lösung nach einem der Ansprüche 1 bis 24, **dadurch gekennzeichnet, dass** die Ergolinverbindung in Form ihres Salzes mit einer Aminosäure aus der Gruppe von Methionin, Tryptophan und Arginin enthalten ist.

26. Stabilisierte wässrige Lösung nach einem der Ansprüche 1 bis 25, **dadurch gekennzeichnet, dass** die Ergolinverbindung in Form ihres Salzes mit einer säurehaltigen Aminosäure aus der Gruppe von Glutaminsäure und Asparaginsäure enthalten ist.

27. Stabilisierte wässrige Lösung nach einem der Ansprüche 1 bis 26, **dadurch gekennzeichnet, dass** die Ergolinverbindung in Form ihres Salzes mit Maleinsäure enthalten ist.

28. Stabilisierte wässrige Lösung nach einem der Ansprüche 1 bis 27, **dadurch gekennzeichnet, dass** die Ergolinverbindung oder deren physiologisch verträgliches Salz oder Derivat in einer Konzentration von 0.01 bis 25.00 mg/mL enthalten ist.

29. Stabilisierte wässrige Lösung nach einem der Ansprüche 1 bis 28, **dadurch gekennzeichnet, dass** die Ergolinverbindung oder deren physiologisch verträgliches Salz oder Derivat in einer Konzentration von 0.25 bis 10.00 mg/mL enthalten ist.

30. Stabilisierte wässrige Lösung nach einem der Ansprüche 1 bis 29, **dadurch gekennzeichnet, dass** die Ergolinverbindung oder deren physiologisch verträgliches Salz oder Derivat in einer Konzentration von 0.50 bis 3.00 mg/mL enthalten ist.

31. Stabilisierte wässrige Lösung nach einem der Ansprüche 1 bis 30, **dadurch gekennzeichnet, dass** die Lösung weiterhin anorganische und/oder organische Verbindungen zur Adjustierung der Osmolarität im Falle einer hypotonen Lösung und/oder zur Adjustierung des pH-Werts umfasst.

32. Stabilisierte wässrige Lösung nach einem der Ansprüche 1 bis 31, **dadurch gekennzeichnet, dass** die Lösung Natriumchlorid zur Adjustierung der Osmolarität umfasst.

33. Stabilisierte wässrige Lösung nach einem der Ansprüche 1 bis 32, **dadurch gekennzeichnet, dass** sie eine Osmolarität von 250 bis 350 mosmol/L aufweist.

34. Stabilisierte wässrige Lösung nach einem der Ansprüche 1 bis 33, **dadurch gekennzeichnet, dass** sie eine Osmolarität von 270 bis 320 mosmol/L aufweist.

35. Stabilisierte wässrige Lösung nach einem der Ansprüche 1 bis 34, **dadurch gekennzeichnet, dass** die Lösung ein Puffersystem aus der Gruppe von Citratpuffer, Carbonatpuffer, Phosphatpuffer oder Maleatpuffer zur Adjustierung des pH-Werts umfasst.

36. Stabilisierte wässrige Lösung nach einem der Ansprüche 1 bis 35, **dadurch gekennzeichnet, dass** die Lösung als Puffersystem einen Citratpuffer umfasst.

37. Stabilisierte wässrige Lösung nach einem der Ansprüche 1 bis 36, **dadurch gekennzeichnet, dass** sie einen pH-Wert im Bereich von 4.00 bis 8.00 aufweisen.

38. Stabilisierte wässrige Lösung nach einem der Ansprüche 1 bis 37, **dadurch gekennzeichnet, dass** sie einen pH-Wert im Bereich von 4.50 bis 7.50 aufweisen.

39. Stabilisierte wässrige Lösung nach einem der Ansprüche 1 bis 38, **dadurch gekennzeichnet, dass** sie einen pH-Wert im Bereich von 5.00 bis 7.00 aufweisen.

40. Verwendung einer stabilisierten wässrigen Lösung nach einem der Ansprüche 1 bis 39 zur Herstellung eines Mittels für die parenterale Behandlung von neurodegenerativen Erkrankungen oder Schädigungen des Gehirns, wobei die stabilisierte wässrige Lösung eine Ergolinverbindung der Formel I, 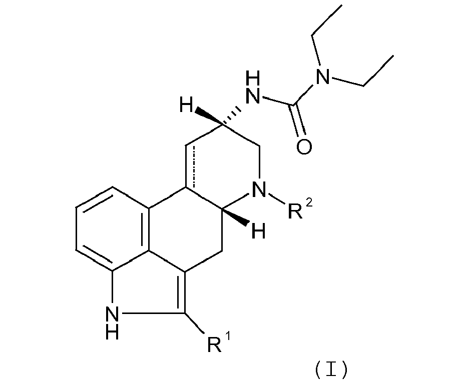 oder deren physiologisch verträgliches Salz oder Derivat, wobei R¹ ein H-Atom oder ein Halogenatom und R² eine Alkyl- oder Alkenylgruppe mit 1 bis 4 Kohlenstoffatomen und ----- eine Einfach- oder Doppelbindung bedeutet, umfasst, welche **dadurch gekennzeichnet ist, dass** die wässrige Lösung weiterhin 0.05 bis 90.00% (m/V) mindestens eines sauerstoffhaltigen Cosolvenz aufweist.

41. Verwendung einer stabilisierten wässrigen Lösung nach einem der Ansprüche 1 bis 40 zur Herstellung eines Mittels für die parenterale Behandlung von neurodegenerativen Erkrankungen oder Schädigungen des Gehirns, **dadurch gekennzeichnet, dass** die parenterale Behandlung subkutan, intramuskulär, intravenös, transdermal oder durch eine in den Blutkreislauf oder das Gewebe implantierte Pumpe erfolgt.

42. Verwendung einer stabilisierten wässrigen Lösung nach einem der Ansprüche 1 bis 41, **dadurch gekennzeichnet, dass** die neurodegenerativen Erkrankungen die Parkinson-Krankheit oder Dystonien sind.

43. Verwendung einer stabilisierten wässrigen Lösung nach einem der Ansprüche 1 bis 42, **dadurch gekennzeichnet, dass** die Schädigung des Gehirns durch einen Schlaganfall oder eine traumatische Gehirnverletzung hervorgerufen wurde.
